# EUROPEAN PATENT SPECIFICATION

(11) **EP 2 667 921 B1**
(45) Date of publication and mention of the grant of the patent: **27.06.2018**
(21) Application number: 12705379.1
(22) Date of filing: 25.01.2012
(51) Int. Cl.: A61M 25/00, A61M 39/22

(54) **CATHETER DRAINAGE ASSEMBLY**
KATHETERDRAINAGEANORDNUNG
ENSEMBLE DRAINAGE PAR CATHÉTER

(30) Priority: 28.01.2011 GB 201101550
(43) Date of publication of application: 04.12.2013
(73) Proprietor: Rocket Medical PLC, Watford Hertfordshire WD2 4XX (GB)
(72) Inventor: WHITFIELD, Paul, Scott, NE13 6HR (GB); VAREY, Richard Martin, WF9 5LY (GB); DAVIS, Beverly, GL52 5JB (GB)
(74) Representative: ip21 Ltd
(86) International application number: PCT/GB2012/000071
(87) International publication number: WO 2012/101405

(56) References cited:
- EP-A1- 0 226 564
- EP-A1- 0 576 380
- WO-A1-03/090843
- WO-A1-2008/144622
- US-A- 4 306 705
- US-A- 5 228 646
- US-A- 5 427 144
- US-A- 5 806 551

## Description

The present invention provides a catheter drainage assembly that can be used as part of a medical device, particularly an indwelling peritoneal catheter.

The use of catheters to drain unwanted fluid or air from a body cavity of a patient is a well-known and frequent medical procedure. Essentially a catheter is a tube which can be located in the cavity of a patient to be treated. Fluid or air can flow along the catheter to outside of the patient, thus alleviating the condition. In most cases, a catheter is a thin, flexible tube ("soft" catheter), though in some uses, it is a larger, solid ("hard") catheter. A catheter left inside the body, either temporarily or permanently, may be referred to as an indwelling catheter.

When used for draining fluid from a body cavity of a patient, catheters are usually employed in conjunction with a drainage bottle such that the fluid drawn from the patient can be safely disposed of.

There are prior known medical devices that can be used with catheters to allow the routine and safe drainage of fluid from a patient, termed catheter drainage assemblies or systems. In use, the passage of fluid from the body cavity can be an uncomfortable, even very painful, process for the patient. In recognition of this, catheter drainage assemblies have been devised that include a flow control valve positioned between the catheter and the drainage bottle, that valve being controllable by the user to regulate the flow of fluid from the cavity to the drainage bottle. In this way, the user can regulate the rate of flow of the fluid, and therefore tailor this according to the degree of discomfort caused.

Prior art documents WO2008/144622, which concerns a bladder drainage aid, and US 4306705, which concerns a slide valve and coupler assembly, are acknowledged.

The present inventors investigated the design of catheter drainage assemblies that can be used to regulate the flow of fluid from a catheter to a drainage bottle. They realised that the existing fluid flow control valves used in such assemblies are prone to becoming clogged with tissue and other body matter in the fluid being drained, or if the fluid is too viscous. This is clearly undesirable since a clogged valve will reduce the efficiency of the catheter drainage assembly. Not only does this lead to unnecessary waste of medical resources, but it can also be inconvenient for the patient since a new drainage assembly has to be connected to the catheter. Upon recognising this problem, the present inventors set about devising an improved catheter drainage assembly. They realised that the fluid flow control valve used in existing catheter drainage assemblies defines a fluid passage between the inlet and outlet ports of that valve, in which any tissue or other body matter in the fluid being drained from the catheter can adhere to the part of the valve used to regulate the flow of the fluid. This adherence causes the blockage in the fluid flow control valve. From this finding, the inventors sought to redesign the fluid flow control valve such that there was a reduced opportunity for any tissue or other body matter in the fluid being drained from the catheter to adhere to the part of the fluid flow control valve used to regulate the flow of the fluid.

Accordingly, a first aspect of the invention provides a catheter drainage assembly as set out in claim 1. Further optional features as set out in subsequent claims. Especially a catheter of the present invention comprising:
a first tube connected to an inlet port of a fluid flow control valve; and, a second tube connected to an outlet port of said fluid flow control valve;
wherein the fluid flow control valve comprises a housing having the inlet and outlet ports; and a displaceable obstructer located in the fluid passage between the inlet and outlet ports of the housing, characterised in that the obstructer comprises an opening, and the obstructer is displaceable in the housing between a first position wherein the opening is not placed in the fluid passage between the inlet and outlet ports, to a second position wherein the opening is placed in the fluid passage between the inlet and outlet ports and wherein the opening comprises a single flow path through the obstructer between opposing sides of the obstructer, wherein the housing comprises a base and wherein there is a single inlet port only and a single outlet port only, wherein the inlet and outlet ports are located on opposing sides of the fluid flow control valve; characterised in that the inlet and outlet ports are located at a staggered distance from the base of the housing, with the inlet port being located further from the base of the housing than the outlet port. Existing catheter drainage assemblies include a fluid flow control valve in which the fluid from the catheter flowed around a displaceable plug in the valve. In flowing around that plug, there is ample opportunity for tissue and or other body matter in the fluid to accumulate on the plug and therefore clog the valve. The present inventors have redesigned the internal flow passage of the fluid flow control valve.

The catheter drainage assembly of the present invention comprises an obstructer with an opening. By "obstructer" we mean the part of the valve which can be used to regulate the flow of fluid from the inlet to outlet ports. That obstructer is displaceable in the housing such that the flow of fluid in the fluid passage between the inlet and outlet ports via the opening can be controlled by the user.

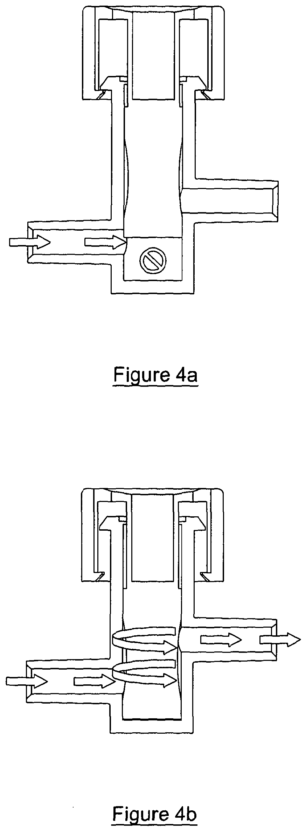

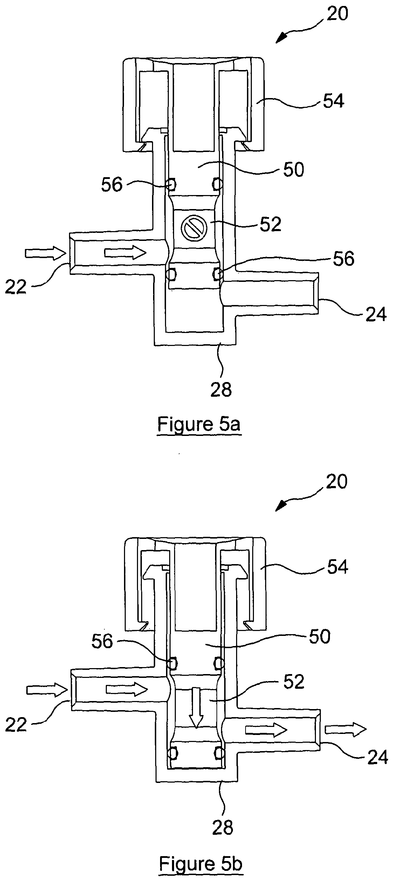

The use of an opening in the obstructer has several advantages. Firstly, the fluid from the catheter flows through an obstructer rather than around a plug. This means there is less opportunity for tissue and or other body matter in the fluid to accumulate, and more viscous liquid can be drawn from the catheter. Hence the fluid flow control valve is less likely to clog when in use. This is further described below and also is depicted in the accompanying figures, particularly Figures 4 and 5. Moreover, as the fluid from the catheter flows through an obstructer rather than around a plug, there is a more direct fluid passage between the inlet and outlet ports of the fluid flow control valve which means that fluid can more rapidly be drained from a catheter. Indeed, the inventors have found that the redesigned fluid flow control valve can increase the flow of the fluid from the catheter by up to 3x that of the existing devices.

It can be appreciated that these improvements to the fluid flow control valve could not have been anticipated from the prior art.

As can be appreciated the type of "fluid" from the catheter that passes through the catheter drainage assembly will vary depending on where the catheter is positioned in the patient. For example, the fluid being drained may be pleural when the catheter is placed in the pleural cavity that surrounds the lungs, i.e. a pleural catheter. The fluid being drained may be peritoneal when the catheter is placed in the peritoneal cavity in the abdomen, i.e. a peritoneal catheter.

The catheter drainage assembly of the present invention can be used with a variety of different catheters, including peritoneal, pleural and urinary catheters. In a preferred embodiment of the invention, the catheter drainage assembly is for use as part of an indwelling peritoneal catheter device.

Preferably the housing is generally cylindrical.

Preferably the actuator is connected to the obstructer, the actuator allowing displacement of the obstructer between the first and second positions. Preferably the obstructer is bias towards the first position. Preferably the obstructer is moveably sealed against the internal wall of the housing. Preferably the seal is a flexible annular moulding, for example an O-ring.

Preferably the first tube is a catheter.

Preferably the catheter drainage assembly is located within an assembly housing. Preferably the assembly housing includes a locking mechanism for reversibly locking the obstructer in the second position. Preferably the assembly housing is generally rectangular and the locking mechanism is a slider located on the upper surface of the assembly housing.

A further aspect of the invention provides a catheter kit comprising:
(i) a catheter drainage assembly of the first aspect of the invention; and
(ii) a drainage bottle connectable to the second tube of the catheter drainage assembly.

Optionally the catheter kit also comprises a catheter. Preferably the catheter kit is an indwelling peritoneal catheter.

A further aspect not forming part of the present invention provides a method of regulating the flow of fluid from a catheter, the method comprising: (i) connecting a catheter drainage assembly of the first aspect of the invention to the catheter and a drainage bottle; then (ii) regulating the flow of fluid from the catheter to the drainage bottle, by displacing the obstructer between a first position wherein the opening is not placed in the fluid passage between the inlet and outlet ports to a second position wherein the opening is placed in the fluid passage between the inlet and outlet ports. The embodiments of the catheter drainage assembly of invention as described above can be used in the catheter drainage assembly of the method of this aspect of the invention. A further aspect of the invention provides a catheter drainage assembly or catheter kit substantially as herein described with reference to the accompanying figures.

All of the features described herein (including any accompanying claims, abstract and drawings), and/or all of the steps of any method or process so disclosed, may be combined with any of the above aspects in any combination, except combinations where at least some of such features and/or steps are mutually exclusive.

The invention will now be further described with reference to the following examples and Figures.
Figure 1: Panel A depicts a fluid flow control valve as known in the art. Panel B depicts an obstructer used in the prior art fluid flow valve assembly with adhered lumps of fibrous tissue and other body fluid matter.
Figure 2: Panel A depicts a catheter drainage assembly according to the present invention located in an assembly housing. The obstructer is positioned in the open position. Panel B also depicts a catheter drainage assembly according to the present invention, with the obstructer positioned in the closed position.
Figure 3: Panel A depicts a fluid flow control valve as used in the catheter drainage assembly according to the present invention. Panel B depicts a detailed view of the obstructer as used in the fluid flow control valve of panel A.
Figure 4: Panels A and B depicts a schematic view of a fluid flow control valve as known in the art in operation. The fluid passage between the inlet and outlet ports is depicted.
Figure 5: Panels A and B depicts a schematic view of a flow control valve as used in the catheter drainage assembly according to the present invention. The fluid passage between the inlet and outlet ports is depicted.

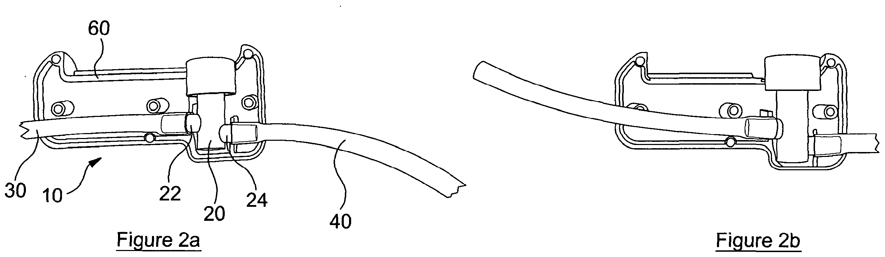

Referring to the drawings and in particular Figures 2, 3 and 5, there is shown a catheter drainage assembly according to the present invention referred to by reference number 10. The catheter drainage assembly 10 of the present invention generally comprises three main parts: a first tube, which can be a catheter or connected to a catheter, a second tube which can be connected to a drainage bottle, and a fluid flow control valve by which the flow of fluid from the first tube to the second tube can be regulated.

By "catheter drainage assembly" we mean that the assembly 10 is constructed of materials and is generally proportioned so as to be suitable for use either directly as a catheter, or can be readily connected to a catheter.

The catheter drainage assembly 10 of the invention is shown in Figure 2 positioned within an assembly housing 60. The assembly housing 60 is an optional feature of the present invention as discussed below. As shown in Figure 2, the catheter drainage assembly 10 comprises a fluid flow control valve 20 having an inlet port 22 and an outlet port 24. A first tube 30 is connected to the inlet port 22 and a second tube 40 is connected to the outlet 24 port of the fluid flow control 20 valve. The catheter drainage assembly 10 has an overall structure and arrangement of features such that it can be used as part of a medical device, for example an indwelling peritoneal catheter. Therefore the catheter drainage assembly 10 can be used in a device which allows fluid to be removed from a body, particularly the body cavity, of a patient in need of such a treatment.

The first tube 30 is a conduit to allow the passage of fluid from the patient to the fluid flow control valve 20. The first tube 30 may be connected to a catheter, in which case suitable connection means may be provided at the distal end of the first tube from the fluid flow control valve 20. In this embodiment, since the first tube 30 is not itself directly a catheter then the first tube can be formed from any suitable material, e.g. a soft plastic material. Suitable connections means by which the first tube 30 may be connected to a catheter are well known in the art. Generally the connection means allow a reversible connection to be made to a catheter.

In an alternative embodiment, the first tube 30 is a catheter such that the distal end of the first tube from the fluid flow control valve 20 can be positioned within the body of a patient. In this embodiment of the invention the first tube 30 is formed from a material suitable for use as a catheter, for example a material with high flexibility and a high level of biocompatibility. Many examples of catheter tubes which can be used as the first tube 30 are well known in the art. They can be formed from, for example, polyurethane, polyethylene, including hydrocoat versions, polyvinylchloride, Teflon(TM), nylon or silicone. Preferably the catheter tube is formed from a medical grade silicone rubber.

The second tube 40 is a conduit to allow the passage of fluid from the fluid flow control valve 20 to a drainage bottle (not shown) when the catheter drainage assembly 10 is part of a device which allows fluid to be removed from a body. The second tube 40 may include suitable connection means at the distal end of the second tube from the fluid flow control valve 20 to allow the tube to be connected to a drainage bottle. The second tube 40 may be formed from any suitable material to allow the passage of fluid from the from the fluid flow control valve 20 to a drainage bottle; for example, a material with high flexibility which allows the user to readily manipulate the second tube 40. Many examples of tubes which can be used as the second tube 40 are well known in the art. They can be formed from, for example, polyurethane, polyethylene, including hydrocoat versions, polyvinylchloride, Teflon(TM), nylon or silicone.

The particular embodiment of the catheter drainage assembly 10 shown in Figure 2 has a single inlet port 22 with associated first tube 30 and a single outlet port 24 with associated second tube 40.

However, it can be appreciated that the catheter drainage assembly 10 of the present invention may be prepared having more than one inlet port 22, for example 2, 3, 4, 5, etc, inlet ports each with an associated first tube 30. Such an embodiment of the invention may be of benefit where the catheter drainage assembly 10 is to be used as part of a medical device that has more than one catheter to be placed in the body of the patient.

Furthermore it can be also be appreciated that the catheter drainage assembly 10 of the present invention may be prepared having more than one outlet port 24, for example 2, 3, 4, 5, etc, outlet ports each with an associated second tube 40. Such an embodiment of the invention may be of benefit where the catheter drainage assembly 10 is to be used as part of a medical device that has more than one drainage bottle. The particular embodiment of the catheter drainage assembly 10 shown in the figures has an arrangement such that the inlet 22 and outlet 24 ports are located on opposing sides of the fluid flow control valve 20. While this may be advantageous for easily allowing the connection of first and second tubes to the fluid flow control valve 20, it can be appreciated that this is arrangement is not essential for the operation of the catheter drainage assembly 10 of the invention.

The particular embodiment of the catheter drainage assembly 10 shown in the figures has an arrangement such that the inlet 22 and outlet 24 ports are located at a staggered distance from the base 28 of the housing 26. The fluid flow control valve 20 provides a means for controlling the rate of flow of fluid through the catheter drainage assembly. As mentioned above, this provides an important level of control for the patient when the catheter drainage assembly is used in a medical device.

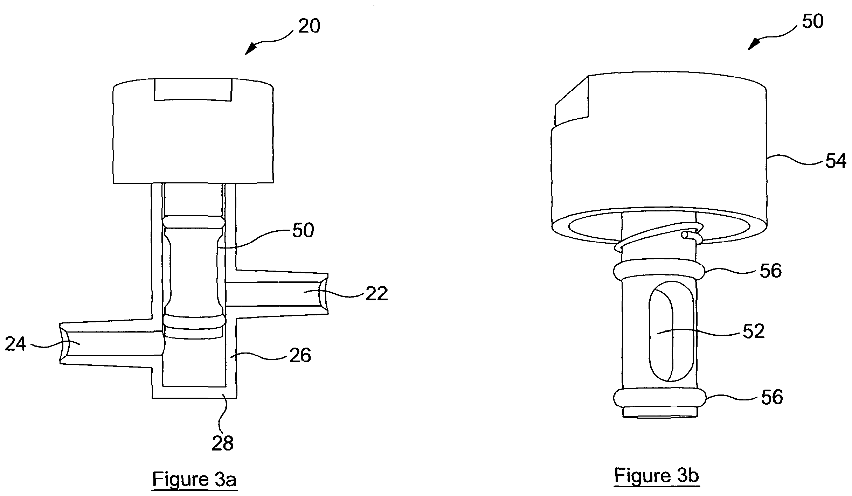

An embodiment of the fluid flow control valve 20 is shown in detail in Figure 3. Here the fluid flow control valve 20 has a single inlet port 22 and a single outlet port 24 positioned on the housing 26. The space between the inlet port 22 and outlet port 24 defines a fluid passage by which fluid entering the inlet port 22 can pass through the fluid flow control valve 20 and exit from the outlet port 24. The housing 26 of the fluid flow control valve 20 can be formed from any suitable material, for example a metal or plastic material. Preferably the housing is formed using rigid polymer, for example a polycarbonate material, and is made according to standard molding procedures as will be well known to the person skilled in the art. The shape of the housing defines a receptacle in which the obstructer is located.

In the embodiment of the invention shown in Figure 3 the housing 26 is generally cylindrical and has a base 28.

An embodiment of the obstructer 50 is shown in detail in Figure 4. The obstructer 50 with an opening 52 is located in the housing 26 fluid flow control valve 20 and can be used by the user of the catheter drainage assembly 10 to control the flow of fluid though the fluid passage. The obstructer 50 is displaceable in the housing 26 between a first position wherein the opening 52 is not placed in the fluid passage between the inlet 22 and outlet 24 ports, to a second position wherein the opening 52 is placed in the fluid passage between the inlet and outlet ports.

Hence when the obstructer 50 is in the first position, though fluid can enter the fluid flow control valve 20 via the inlet port 22, there is no passage through the flow control valve 20 for that fluid. Therefore the fluid will not continue to flow through the catheter. Thus the fluid flow control valve 20 is "closed" when the obstructer 50 is in the first position.

However, on displacing the obstructer 50 through to the second position, the opening 52 is brought in to engagement with the fluid passage between the inlet 22 and outlet 24 ports. Fluid can then flow through the fluid flow control valve 20 from the inlet 22 to outlet 24 port. Thus the fluid flow control valve 20 is "open" when the obstructer 50 is in the second position.

For the avoidance of doubt, the obstructer 50 can be displaced from the first position to the second position, and stopped at any point in that displacement process. Therefore, while in the first position none of the opening 52 of the obstructer 50 is in engagement with the fluid passage, the user of a medical device incorporating the catheter drainage assembly 10 of the invention can displace the obstructer 50 such that a varying amount of the opening 52 is in engagement with the fluid passage. By increasing the amount of the opening 52 in engagement with the fluid passage, then more of the fluid passage becomes open to the flow of fluid, and hence the rate of flow of the fluid through the catheter drainage assembly 10 can be controlled. This means for controlling the rate of flow of fluid through the catheter drainage assembly 10 provides an important level of control for the patient when the catheter drainage assembly 10 is used in a medical device.

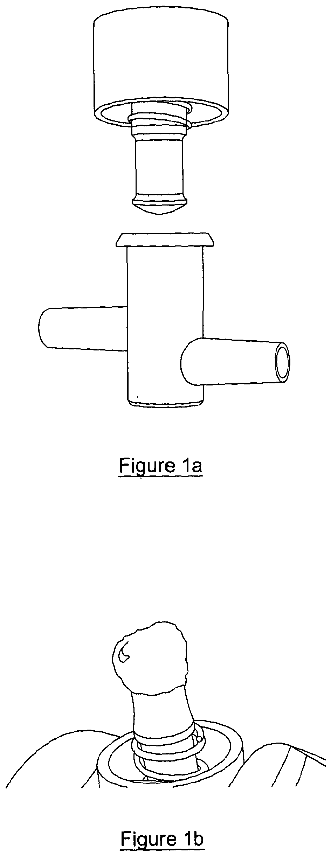

In contrast to the obstructer used in the prior art, an example of which is shown in Figure 1B, the use of an opening 52 in the obstructer 50 provides a more direct fluid passage from the inlet 22 and outlet 24 ports. A schematic illustration of the flow of fluid through the prior art fluid flow control valve is shown in Figure 4, while the flow of fluid through the fluid flow control valve used in the catheter drainage assembly 10 of the present invention is shown in Figure 5. When used in the catheter drainage assembly 10 of the present invention, this provides a number of clear and surprising benefits.

Firstly by using an obstructer 50 with an opening 52 the fluid flow control valve 20 is exposed to the fluid for a shorter period of time than the prior art fluid flow control valve. Furthermore, the obstructer 50 with an opening 52 allows the fluid to flow through the obstructer 50, rather than round the obstructer used in the prior art fluid flow control valve and hence a smaller surface area of the obstructer 50 is exposed to the fluid.

This has the effect that by using obstructer 50 with an opening 52, the fluid flow control valve 20 is much less likely to have a build up of tissue and other material in the fluid. Hence there is a reduction in the likelihood of the flow control valve 20 to be blocked by a build up of such tissue.

Moreover, by using obstructer 50 with an opening 52, the fluid flow control valve 20 has a more direct fluid passage from the inlet 22 and outlet 24 ports. Therefore the flow of fluid through the fluid flow control valve 20, and hence the catheter drainage assembly 10, is faster and therefore when used in a medical device more fluid can be drained from a patient in a set period of time.

The obstructer 50 can be any size or shape, with the proviso that it can be located in the housing 26 of the fluid flow control valve 20. As can be appreciated, the size and shape of the obstructer 50 will often be determined to a large extent by the size and shape of the obstructer housing 26 of the fluid flow control valve 20. In the embodiment of the invention illustrated in the accompanying figures, the fluid flow control valve 20 and the housing 26 is generally of a cylindrical shape, and the obstructer 50 also has a cylindrical shape having a slightly smaller diameter than that of the housing, thus allowing the obstructer 50 to be located therein.

For the avoidance of doubt, by "opening" we mean that there is a hole through the body of the obstructer 50 which allows fluid to flow through obstructer 50 in a controllable manner. The opening 52 of the obstructer 50 can be any shape or size that allows it to be used to control the flow of fluid though the fluid passage.

The obstructer 50 can be displaced in a number different ways so as to allow the opening 52 to engage with the direct fluid passage from the inlet 22 and outlet 24 ports. In the embodiment of the invention illustrated in the accompanying figures, the obstructer 50 can be vertically displaced with reference to the housing 26 of the fluid flow control valve 20. That displacement can be a "push-pull" action, or it can also be combined with some rotation.

The obstructer 50 is connected to an actuator 54 that allows displacement of the obstructer 50 between the first and second positions as discussed above. The actuator 54 can be readily operated by the user of the catheter drainage assembly 10. In the embodiment of the present invention illustrated in the accompanying figures, the actuator 54 is positioned at one end of the obstructer 50 (that distal to the base 28 of the housing 26) and is sized so as to cover the housing 26 of the fluid flow control valve 20.

As can be appreciated by the skilled person, there are a number of different means by which the actuator 54 can be connected to the obstructer 50 so as to allow displacement of the between the first and second positions. However, it is preferred that the actuator 54 is permanently connected to the obstructer 50, such that by pushing or pulling the actuator 54, possibly combined with some rotational force, the obstructer 50 can be displaced from the first position to the second position, and stopped at any point in that displacement process.

Preferably the actuator 54 is formed from the same material as the obstructer 50; more preferably the obstructer 50 and actuator 54 are different regions of a single element. The actuator 54 and obstructer 50 can be formed from a suitable material, for example a metal or plastic material. Preferably the obstructer 50 and actuator 54 is formed using rigid polymer, for example a polycarbonate material, and is made according to standard molding procedures as will be well known to the person skilled in the art.. More preferably the obstructer 50 and actuator 54 is formed of a material that is not amenable for the build up of tissue and other material in the fluid. The obstructer 50 is displaceable in the housing 26 between a first position wherein the opening 52 is not placed in the fluid passage between the inlet 22 and outlet 24 ports, to a second position wherein the opening 52 is placed in the fluid passage between the inlet and outlet ports. It can be appreciated by the skilled person that the obstructer 50 should be located in the housing 26 such that there is a seal between the obstructer 50 and the internal wall of the housing 26 so that fluid cannot pass from the inlet 22 and outlet 24 ports except via the opening 52 in the obstructer 50. Since the obstructer 50 has to be displaceable in the housing 26, then that seal should also be displaceable.

While there are a number of different means by which this seal can be formed, in the embodiment of the invention illustrated in the accompanying figures the seal is a flexible annular moulding 56, for example an O-ring; preferably the flexible annular moulding 56 is positioned on the obstructer 50 at either end of the opening 52. As can be appreciated, the flexible annular moulding 56 will therefore prevent the flow of fluid around the sides of the obstructer 50 and will also prevent the flow of fluid from the open end of the housing 26. In an embodiment of the invention, the obstructer 50 is bias towards the first position, i.e. the opening 52 is not in engagement with the fluid flow passage. This means that the user has to actively engage with the catheter drainage assembly 10 to allow the flow of fluid from the first tube to the second tube. In this way, the flow of fluid to be removed from the body cavity of a patient can be directly controlled and dictated by the patient so as to provide maximum comfort to them while performing this action.

As can be appreciated, while the obstructer 50 should be displaceable in the fluid flow control valve 20 it is undesirable for the obstructer 50 to exit from the housing 26 since this can result in the uncontrolled flow of fluid from the first tube. It can be appreciated that there are a number of different means by which the obstructer 50 may be retained in position. As shown in Figure 5 in one embodiment of the invention the obstructer 50 can be held in place in the housing 26 of the fluid flow control valve by forming the housing 26 and the actuator 54 with flanges that engage to prevent the obstructer 50 from being fully removed from the housing 26.

An optional feature of the catheter drainage assembly 10 of the present invention is the assembly housing 60, as shown in Figure 2. In use the catheter drainage assembly 0 is positioned within that assembly housing 60. The assembly housing 60 can provide a number of advantageous benefits and features to the catheter drainage assembly 10.
Firstly, the assembly housing 60 can generally provide a protective function to the catheter drainage assembly 10, i.e. to prevent dust or dirt building up on the catheter drainage assembly 10 which may impair its function. It can also prevent accidental damage to the catheter drainage assembly 10, and optionally also provide guides that may retain the first 30 and second 40 tubes in position so as to reduce the likelihood of them disengaging with the input 24 and output 26 ports.

The assembly housing 60 can also be provided with features to aid in the operation of the catheter drainage assembly 10. For example, the assembly housing 60 can include a locking mechanism for reversibly locking the obstructer 50 in the second position. Hence if the user of catheter wishes to retain the fluid flow control valve in the "open" position for a period of time, they can operate the locking mechanism for this effect. Though not shown in the accompanying figures, an embodiment of the locking mechanism is a slider located on the upper surface of the assembly housing. In use, the slider can be moved across the upper surface of the actuator 54, to hold the obstructer 50 in the second position.
The assembly housing 60 can be formed from a suitable material, for example a metal or plastic material. Preferably assembly housing 60 is formed using a polycarbonate material and is made according to standard molding procedures as will be well known to the person skilled in the art.

The catheter drainage assembly 10 discussed above and shown in the accompanying figures can be manufactured and supplied as part of a catheter kit, which can further comprise a drainage bottle connectable to the second tube of the catheter drainage assembly, along with optionally instructions for use. "Drainage bottles" are well known in the art. Preferably the drainage bottle is a vacuum bottle. This can aid the flow of fluid to the drainage bottle since this draws the fluid from the body through the catheter drainage assembly 10. As can be appreciated the catheter drainage assembly of the invention can be incorporated in to a variety of different catheter kits, including peritoneal, pleural and urinary catheters. Preferably the catheter kit is an indwelling peritoneal catheter.

As way of example, there follows a brief discussion of the operation of an embodiment of the catheter drainage assembly 10 of the present invention.

The catheter drainage assembly 10 is provided as part of a catheter kit and is located in an assembly housing 60. The first tube 30 is connected to a catheter that has been positioned within the body of a patient, e.g. the peritoneal cavity. A drainage bottle is also connected to the second tube 40. The user then regulates the flow of fluid from the catheter to the drainage bottle, by manipulating the actuator 54 as discussed above. If required, the user can lock the fluid flow control valve in the "open position" using the actuator to displace the obstructer 50 to the second position, and then sliding the locking mechanism located on the upper surface of the assembly housing. If a change in drainage bottle is needed, then the user can displace the fluid flow control valve 20 to the "closed" position using the actuator to displace the obstructer 50 to the first position. In this way, fluid does not flow through the fluid flow control valve and the drainage bottle can be detached from the second tube 40 and a new drainage bottle attached.

A further aspect of the invention provides a catheter drainage assembly or catheter kit substantially as herein described with reference to the accompanying figures, in particular Figures 2, 3 and 5.

It can be understood that the present invention is not limited to the particular embodiment described herein.

## Claims

1. A catheter drainage assembly (10) comprising:
a first tube (30) connected to an inlet port (22) of a fluid flow control valve (20); and, a second tube (40) connected to an outlet port (24) of said fluid flow control valve;
wherein the fluid flow control valve comprises a housing (26) having the inlet and outlet ports; and a displaceable obstructer (50) located in the fluid passage between the inlet and outlet ports of the housing, wherein the obstructer comprises an opening (52),
and the obstructer is displaceable in the housing between a first position wherein the opening (52) is not placed in the fluid passage between the inlet (22) and outlet (24) ports, to a second position wherein the opening is placed in the fluid passage between the inlet and outlet ports and wherein the opening (52) comprises a single flow path through the obstructer between opposing sides of the obstructer, wherein the housing (26) comprises a base (28) and wherein there is a single inlet port only (22) and a single outlet port only (24), wherein the inlet and outlet ports are located on opposing sides of the fluid flow control valve (20); **characterised in that** the inlet and outlet ports are located at a staggered distance from the base (28) of the housing (26), with the inlet port (22) being located further from the base (28) of the housing (26) than the outlet port (24).

2. The catheter drainage assembly (10) according to claim 1 wherein the housing (26) is generally cylindrical.

3. The catheter drainage assembly (10) according to claim 1 or 2 wherein an actuator (54) is connected to the obstructer (50), the actuator (54) allowing displacement of the obstructer (50) between the first and second positions.

4. The catheter drainage assembly (10) according to any of the previous claims wherein the obstructer (50) is bias towards the first position.

5. The catheter drainage assembly (10) according to any of the previous claims wherein the obstructer (50) is moveably sealed against the internal wall of the housing (26).

6. The catheter drainage assembly (10) according to claim 5 wherein the seal is a flexible annular moulding.

7. The catheter drainage assembly (10) according to any of the previous claims wherein the first tube (30) is a catheter.

8. The catheter drainage assembly (10) according to any of the previous claims wherein the assembly (10) is located within an assembly housing (60).

9. The catheter drainage assembly (10) according to claim 8 wherein the assembly housing (60) includes a locking mechanism for reversibly locking the obstructer (50) in the second position.

10. The catheter drainage assembly (10) according to claim 9 wherein the assembly housing (60) is generally rectangular and the locking mechanism is a slider located on the upper surface of the assembly housing (60).

11. A catheter kit comprising:
(i) a catheter drainage assembly (10) as defined in any of the previous claims; and
(ii) a drainage bottle connectable to the second tube (40) of the catheter drainage assembly.

12. The catheter kit of claim 11 wherein the catheter kit is an indwelling peritoneal catheter.

## Patentansprüche

1. Katheterdrainagebaugruppe (10), umfassend:
einen ersten Schlauch (30), der mit einem Einlassanschluss (22) eines Fluiddurchflussregelventils (20) verbunden ist; und einen zweiten Schlauch (40),
der mit einem Auslassanschluss (24) des Fluiddurchflussregelventils verbunden ist; wobei das Fluiddurchflussregelventil ein Gehäuse (26) umfasst,
das die Ein- und Auslassanschlüsse aufweist; und ein verschiebbares Sperrteil (50), das sich in der Fluidpassage zwischen den Ein- und Auslassanschlüssen des Gehäuses befindet, wobei das Sperrteil eine Öffnung (52) umfasst und das Sperrteil in dem Gehäuse verschiebbar ist zwischen einer ersten Position, in welcher die Öffnung (52) nicht in der Fluidpassage zwischen dem Einlassanschluss (22) und dem Auslassanschluss (24) positioniert ist, und einer zweiten Position, in welcher die Öffnung in der Fluidpassage zwischen dem Einlassanschluss und dem Auslassanschluss positioniert ist, und wobei die Öffnung (52) einen einzigen Durchflussweg durch das Sperrteil hindurch zwischen gegenüberliegenden Seiten des Sperrteils umfasst, wobei das Gehäuse (26) eine Basis (28) umfasst und wobei es nur einen einzigen Einlassanschluss (22) und nur einen einzigen Auslassanschluss (24) gibt, wobei sich die Einlass- und Auslassanschlüsse an gegenüberliegenden Seiten des Fluiddurchflussregelventils (20) befinden;
**dadurch gekennzeichnet, dass** sich die Einlass- und Auslassanschlüsse in einer gestaffelten Entfernung von der Basis (28) des Gehäuses (26) befinden,
wobei sich der Einlassanschluss (22) weiter entfernt von der Basis (28) des Gehäuses (26) befindet als der Auslassanschluss (24).

2. Katheterdrainagebaugruppe (10) nach Anspruch 1, wobei das Gehäuse (26) im Allgemeinen zylindrisch ist.

3. Katheterdrainagebaugruppe (10) nach Anspruch 1 oder 2, wobei mit dem Sperrteil (50) ein Stellglied (54) verbunden ist und das Stellglied (54) die Verschiebung des Sperrteils (50) zwischen der ersten und der zweiten Position ermöglicht.

4. Katheterdrainagebaugruppe (10) nach einem der vorstehenden Ansprüche, wobei das Sperrteil (50) in Richtung der ersten Position vorgespannt ist.

5. Katheterdrainagebaugruppe (10) nach einem der vorstehenden Ansprüche, wobei das Sperrteil (50) bewegbar gegen die Innenwand des Gehäuses abgedichtet ist.

6. Katheterdrainagebaugruppe (10) nach Anspruch 5, wobei die Dichtung ein flexibles ringförmiges Formteil ist.

7. Katheterdrainagebaugruppe (10) nach einem der vorstehenden Ansprüche, wobei der erste Schlauch (30) ein Katheter ist.

8. Katheterdrainagebaugruppe (10) nach einem der vorstehenden Ansprüche, wobei sich die Baugruppe (10) im Inneren eines Baugruppengehäuses (60) befindet.

9. Katheterdrainagebaugruppe (10) nach Anspruch 8, wobei das Baugruppengehäuse (60) einen Verriegelungsmechanismus zum reversiblen Verriegeln des Sperrteils (50) in der zweiten Position enthält.

10. Katheterdrainagebaugruppe (10) nach Anspruch 9, wobei das Baugruppengehäuse (60) im Allgemeinen rechteckig ist und der Verriegelungsmechanismus ein Schieber ist, der sich auf der Oberseite des Baugruppengehäuses (60) befindet.

11. Katheter-Kit, umfassend:
(i) eine Katheterdrainagebaugruppe (10) entsprechend der Definition in den vorstehenden Ansprüchen; und
(ii) eine Drainageflasche, die mit dem zweiten Schlauch (40) der Katheterdrainagebaugruppe verbunden werden kann.

12. Katheter-Kit nach Anspruch 11, wobei das Katheter-Kit ein Peritonealdauerkatheter ist.

## Revendications

1. Un ensemble de drainage par cathéter (10) comprenant :
un premier tube (30) raccordé à un orifice d'entrée (22) d'une soupape de contrôle d'écoulement de fluide (20) ; et un deuxième tube (40) raccordé à un orifice de sortie (24) de ladite soupape de contrôle d'écoulement de fluide ; la soupape de contrôle d'écoulement de fluide comprenant un boîtier (26) ayant les orifices d'entrée et de sortie ; et un obturateur déplaçable (50) situé dans le passage de fluide entre les orifices d'entrée et de sortie du boîtier, l'obturateur comprenant une ouverture (52), et
l'obturateur étant déplaçable dans le boîtier entre une première position dans laquelle l'ouverture (52) n'est pas placée dans le passage de fluide entre les orifices d'entrée (22) et de sortie (24), et une deuxième position dans laquelle l'ouverture est placée dans le passage de fluide entre les orifices d'entrée et de sortie et l'ouverture (52) comprenant un trajet d'écoulement unique à travers l'obturateur entre des côtés opposés de l'obturateur, le boîtier (26) comprenant une base (28) et dans lequel il y a un orifice d'entrée unique seulement (22) et un orifice de sortie unique seulement (24), les orifices d'entrée et de sortie étant situés sur des côtés opposés de la soupape de contrôle d'écoulement de fluide (20) ; **caractérisé en ce que** les orifices d'entrée et de sortie sont situés à une distance décalée de la base (28) du boîtier (26), l'orifice d'entrée (22) étant situé plus loin de la base (28) du boîtier (26) que l'orifice de sortie (24).

2. L'ensemble de drainage par cathéter (10) selon la revendication 1 dans lequel le boîtier (26) est généralement cylindrique.

3. L'ensemble de drainage par cathéter (10) selon la revendication 1 ou la revendication 2 dans lequel un actionneur (54) est raccordé à l'obturateur (50), l'actionneur (54) permettant le déplacement de l'obturateur (50) entre les première et deuxième positions.

4. L'ensemble de drainage par cathéter (10) selon n'importe lesquelles des revendications précédentes dans lequel l'obturateur (50) est sollicité en direction de la première position.

5. L'ensemble de drainage par cathéter (10) selon n'importe lesquelles des revendications précédentes dans lequel l'obturateur (50) est scellé de façon mobile contre la paroi interne du boîtier (26).

6. L'ensemble de drainage par cathéter (10) selon la revendication 5 dans lequel le scellement est un moulage annulaire souple.

7. L'ensemble de drainage par cathéter (10) selon n'importe lesquelles des revendications précédentes dans lequel le premier tube (30) est un cathéter.

8. L'ensemble de drainage par cathéter (10) selon n'importe lesquelles des revendications précédentes, l'ensemble (10) étant situé à l'intérieur d'un boîtier (60) d'ensemble.

9. L'ensemble de drainage par cathéter (10) selon la revendication 8 dans lequel le boîtier (60) d'ensemble inclut un mécanisme de verrouillage pour verrouiller de façon réversible l'obturateur (50) dans la deuxième position.

10. L'ensemble de drainage par cathéter (10) selon la revendication 9 dans lequel le boîtier (60) d'ensemble est généralement rectangulaire et le mécanisme de verrouillage est un dispositif coulissant situé sur la surface supérieure du boîtier (60) d'ensemble.

11. Un kit de cathéter comprenant :
(i) un ensemble de drainage par cathéter (10) tel que défini dans n'importe lesquelles des revendications précédentes ; et
(ii) une bouteille de drainage pouvant être raccordée au deuxième tube (40) de l'ensemble de drainage par cathéter.

12. Le kit de cathéter de la revendication 11, le kit de cathéter étant un cathéter péritonéal à demeure.
